# EUROPÄISCHE PATENTSCHRIFT

(11) **EP 1 296 654 B1**
(45) Veröffentlichungstag und Bekanntmachung des Hinweises auf die Patenterteilung: **02.08.2006**
(21) Anmeldenummer: 01953861.0
(22) Anmeldetag: 29.06.2001
(51) Int. Cl.: A61K 9/20, A61K 9/48

(54) **VERWENDUNG VON DICALCIUMPHOSPHATANHYDRID-PULVER**
USE OF DICALCIUM PHOSPHATE ANHYDRIDE POWDER
UTILISATION DE DICALCIUM PHOSPHATE ANHYDRIDE EN POUDRE

(30) Priorität: 04.07.2000 DE 10032434
(43) Veröffentlichungstag der Anmeldung: 02.04.2003
(73) Patentinhaber: Chemische Fabrik Budenheim KG, 55257 Budenheim (DE)
(72) Erfinder: FISCHER, Erhard, D-55218 Ingelheim (DE); SCHEUER, Gerhard, 55268 Nieder-Olm (DE); MEVEN, Jürgen, 55126 Mainz (DE); MALLMANN, Stefan, 55262 Heidesheim (DE); JOHN, Silke geb. Kratzmann, 55262 Mainz-Kastel (DE)
(74) Vertreter: Weber, Dieter
(86) Internationale Anmeldenummer: PCT/DE2001/002484
(87) Internationale Veröffentlichungsnummer: WO 2002/002082

(56) Entgegenhaltungen:
- EP-A- 0 210 661
- EP-A- 0 644 156
- WO-A-98/50019
- DE-A- 4 122 960
- US-A- 4 675 188
- US-A- 4 721 615

## Beschreibung

In der pharmazeutischen Industrie wird in großem Umfang als Trägersubstanz Dicalciumphosphatdihydrat verwendet, doch ist der Anwendungsbereich hiervon auf Wirkstoffe beschränkt, die nicht wasserempfindlich sind, da das Dihydrat unter ungünstigen Bedingungen bereits ab 40 °C langsam Wasser abspalten kann. Als Trägersubstanz für Arzneimittelformen, insbesondere für die Direkttablettierung pharmazeutischer Produkte, gewann daher die Verwendung von Dicalciumphosphatanhydrid zunehmendes Interesse.

Dicalciumphosphatanhydrid wird im allgemeinen durch Neutralisation von Phosphorsäure mit einer basischen Calciumverbindung, z. B. Calciumhydroxid oder Calciumcarbonat, bei einer Temperatur oberhalb 60 °C hergestellt. Bei niedrigeren Temperaturen entsteht Dicalciumphosphatdihydrat bzw. ein Gemisch von Dicalciumphosphatdihydrat und -anhydrid. Das Dicalciumphosphatanhydrid entsteht im allgemeinen in der Form feiner Kristalle, die in Mühlen zu Pulver vermahlen werden. Die so erhaltenen Produkte haben üblicherweise eine durchschnittliche Komgröße kleiner als 20 µm und ein Schüttgewicht von 500 bis 900 g/l. Wegen der hohen Feinheit und der damit verbundenen schlechten Rieselfähigkeit sind diese Anhydridprodukte nicht direkt tablettierbar, sondem müssen vor der Anwendung granuliert werden, um eine ausreichende Fließfähigkeit zu gewähneisten, die für eine gleichmäßige Befüllung der Matrizen der Tablettiermaschinen erforderlich ist.

Um diese Nachteile zu beseitigen, ging man dazu über, grobkörniges Dicalciumphosphatanhydrid für eine Direkttablettierung zu verwenden. Beispielsweise beschreibt die US-A-4 707 361 ein grobkörniges Dicalciumphosphatanhydrid-Pulver für die Tablettierung mit einer Komgröße von mindestens 80 % größer als etwa 44 µm und wenigstens 95 % kleiner als etwa 420 µm sowie mit einer spezifischen Oberfläche größer als 5 m²/g. Dieses Pulver wird durch Dehydratisierung von Dicalciumphosphatdihydrat hergestellt.

Die DE-A-4 122 960 beschreibt ein Verfahren zur Herstellung von grobkörnigem Dicalciumphosphatanhydrid-Pulver, bei dem mindestens 95 % eine Korngröße über 45 µm haben und dessen mittlerer Korndurchmesser im Bereich von zwischen 130 und 150 µm liegt. Dieses für die Direkttablettierung vorgesehene Pulver wird in der Weise hergestellt, daß verdünnte Phosphorsäure bei einer Temperatur von 70 bis 90 °C vorgelegt wird und Kalkmilch und weitere Phosphorsäure unter Einhaltung eines pH-Wertes von 3 bis 4,5 zugeführt werden. Anschließend wird der pH-Wert mit Kalkmilch auf 5,5 bis 6,8 eingestellt.

Schließlich beschreibt die EP-A-0 644 156 für Arzneimittel, Kosmetika und Nahrungsmittel ein Dicalciumphosphat mit geringem Hydratwassergehalt und einer spezifischen Oberfläche von 20 bis 60 m²/g, einem Schüttgewicht von maximal 200 g/I und einer Korngröße der Primärpartikel von 0,1 bis 5 µm und einer solchen der Agglomerate von 2 bis 10 µm. Die Herstellung erfolgt durch Fällung aus Phosphorsäure und einer alkalischen Calciumverbindung unter Anwesenheit einer organischen komplexierenden Verbindung sowie Granulierung durch Sprühtrocknung.

Die der Erfindung zugrundeliegende Aufgabe bestand nun darin, ein Dicalciumphosphatanhydrid-Pulver mit überlegenem Rieselverhalten zu bekommen, mit Hilfe dessen insbesondere bei der Direkttablettierung pharmazeutischer Präparate erhöhte Calciummengen in das Präparat gebracht werden können.

Erfindungsgemäß verwendet man Dicalciumphosphatanhydrid-Pulver, von dem mindestens 50 Gew.-% eine Komgröße zwischen 45 und 150 µm, maximal 50 Gew.-% eine Komgröße von <45 µm und maximal 5 Gew.-% eine Korngröße >150 µm haben und welches ein Schüttgewicht von 1000 bis 1500 g/l und eine spezifische Oberfläche von <5 m²/g besitzt, zur Direkttablettierung oder Kapselfüllung pharmazeutischer Präparate.

Vorzugsweise verwendet man ein Dicalciumphosphatanhydrid-Pulver mit einem Schüttgewicht von 1200 bis 1400 g/I, bevorzugt ein solches Pulver mit einer spezifischen Oberfläche <2 m²/g.

Das erfindungsgemäß verwendete Dicalciumphosphatanhydrid-Pulver zeichnet sich durch ein besonders hohes Schüttgewicht und trotz der relativ hohen Feinheit durch ein sehr gutes Rieselverhalten aus. Aus diesem Grund eignet sich dieses Pulver besonders für die Direkttablettierung ohne vorausgehende Granulierung und bringt in das erhaltene Präparat eine größere Menge Calcium als andere Anhydridpulver ein, ohne daß das Volumen der Arzneimittelform stark zunimmt. Das Produkt eignet sich auch hervorragend zum Befüllen von Hart- und Weichgelatinekapseln.

Die Komgrößenverteilung der erfindungsgemäß verwendeten Dicalciumphosphatanhydrid-Pulver kann durch rein mechanische Verfahren durch Absieben von Fraktionen bestimmter Komgrößen und gezieltes Zusammenmischen bestimmter Anteile verschiedener Komgrößenfraktionen erhalten werden. Die oben angeführten Eigenschaften des erfindungsgemäß verwendeten Dicalciumphosphatanhydrid-Pulvers können aber auch durch geeignete Reaktionsführung eingestellt werden.

### Beispiele

### Beispiel 1 und Vergleichsbeispiel 1

Die beiden Testformulierungen bestanden aus jeweils 99 Gew.-% Dicalciumphosphatanhydrid und 1 Gew.-% Magnesiumstearat.

Das erfindungsgemäß verwendete Dicalciumphosphatanhydrid hatte folgende Eigenschaften:

| | |
|---|---|
| Schüttgewicht | 1300 g/l |
| spezifische Oberfläche | 1,5 m²/g |

| Siebanalyse: | |
|---|---|
| durch 45 µm: | 35 Gew.-% |
| 45-150 µm: | 63 Gew.-% |
| über 150 µm: | 2 Gew.-% |

Das Dicalciumphosphatanhydrid gemäß Vergleichsbeispiel hatte folgende Eigenschaften:

| | |
|---|---|
| Schüttgewicht | 750 g/l |
| spezifische Oberfläche | 15 m²/g |

| Siebanalyse: | |
|---|---|
| durch 45 µm: | 3 Gew.-% |
| 45-150 µm: | 42 Gew.-% |
| über 150 µm: | 55 Gew.-% |

Aus beiden Testformulierungen wurden Tabletten mit folgenden Tablettierparametern gepreßt:

| | |
|---|---|
| Preßkraft | 40 kN |
| Tablettiergeschwindigkeit | 20000 Tabletten/h |
| Tablettendurchmesser: | 10 mm |
| Füllhöhe der Matrizen: | so eingestellt, daß eine Tablettendicke von 3,0 mm erreicht wird. |

**Ergebnis:**

| | Formulierung gemäß der Erfindung | Formulierung gemäß Vergleichsbeispiel |
|---|---|---|
| Tablettengewicht | 515 mg | 440 mg |
| mg Ca/Tablette | 150 mg | 128 mg |
| Tablettendicke | 3,0 mm | 3,0 mm |
| Tablettenhärte | 52 N | 112 N |

Bei gleicher Tablettendicke bekommt man bei Verwendung des erfindungsgemäßen Dicalciumphosphatanhydrid-Pulvers einen erheblich höheren Calciumgehalt pro Tablette.

### Beispiel 2 und Vergleichsbeispiel 2

Die beiden Testformulierungen bestanden aus jeweils 60 Gew.% Dicalciumphosphatanhydrid, 39 Gew.% MCC (mikrokristalline Zellulose) und 1 Gew.-% Magnesiumstearat.

Das erfindungsgemäß verwendete Dicalciumphosphatanhydrid hatte folgende Eigenschaften:

| | |
|---|---|
| Schüttgewicht | 1300 g/l |
| spezifische Oberfläche | 1,5 m²/g |

| Siebanalyse: | |
|---|---|
| durch 45 µm: | 35 Gew.-% |
| 45-150 µm: | 63 Gew.-% |
| über 150 µm: | 2 Gew.% |

Das im Vergleichsbeispiel verwendete Dicalciumphosphatanhydrid hatte folgende Eigenschaften:

| | |
|---|---|
| Schüttgewicht | 750 g/l |
| spezifische Oberfläche | 15 m²/g |

| Siebanalyse: | |
|---|---|
| durch 45 µm: | 3 Gew.-% |
| 45-150 µm: | 42 Gew.% |
| über 150 µm: | 55 Gew.-% |

Aus beiden Testformulierungen wurden Tabletten mit folgenden Tablettierparametern gepreßt

| | |
|---|---|
| Preßkraft | 40 kN |
| Tablettiergeschwindigkeit | 20000 Tabletten/h |
| Tablettendurchmesser | 10 mm |
| Füllhöhe der Matrizen: | so eingestellt, daß ein Tablettengewicht von 325 mg erreicht wurde. |

**Ergebnis:**

| | Formulierung gemäß der Erfindung | Formulierung gemäß Vergleichsbeispiel |
|---|---|---|
| Tablettengewicht | 325 mg | 325 mg |
| mg Ca/Tablette | 77 mg | 77 mg |
| Tablettendicke | 2,2 mm | 2,5 mm |
| Tablettenhärte | 134 N | 123 N |

Bei gleicher Calciummenge in den beiden Tabletten hatte die unter Verwendung des erfindungsgemäß eingesetzten Dicalciumphosphatanhydrid-Pulvers eine erheblich geringere Tablettendicke.

## Patentansprüche

1. Verwendung von Dicalciumphosphatanhydrid-Pulver, von dem mindestens 50 Gew.% eine Korngröße zwischen 45 und 150 µm, maximal 50 Gew.-% eine Korngröße von <45 µm und maximal 5 Gew.% eine Korngröße von >150 µm haben und welches ein Schüttgewicht von 1000 bis 1500 g/l und eine spezifische Oberfläche von <5 m²/g besitzt, zur Direkttablettierung oder Kapselfüllung pharmazeutischer Präparate.

2. Verwendung von Dicalciumphosphatanhydrid-Pulver mit einem Schüttgewicht von 1200 bis 1400 g/l nach Anspruch 1.

3. Verwendung von Dicalciumphosphatanhydrid-Pulver mit einer spezifischen Oberfläche <2 m²/g nach Anspruch 1 oder 2.

## Claims

1. Use of dicalcium phosphate anhydride powder of which at least 50% by weight is of a grain size of between 45 and 150 µm, a maximum of 50% by weight is of a grain size of < 45 µm and a maximum of 5% by weight is of a grain size > 150 µm and which has a bulk weight of between 1000 and 1500 g/l and a specific surface area of < 5m²/g, for the direct tabletting or capsule filling of pharmaceutical preparations.

2. Use of dicalcium phosphate anhydride powder with a bulk weight of between 1200 and 1400 g/l as set forth in claim 1.

3. Use of dicalcium phosphate anhydride powder with a specific surface area <2 m²/g as set forth in claim 1 or claim 2.

## Revendications

1. Utilisation de poudre de phosphate dicalcique anhydre dont au moins 50% en poids ont une dimension comprise entre 45 et 150 µm, dont au maximum 50% en poids ont une dimension inférieure à 45 µm et dont au maximum 5% en poids ont une dimension supérieure à 150 µm et qui présente une densité apparente de 1000 à 1500 g/l et une surface spécifique inférieure à 5m²/g, pour mettre des préparations pharmaceutiques directement sous la forme de comprimés ou pour les encapsuler.

2. Utilisation de poudre phosphate dicalcique anhydre ayant une densité apparente de 1200 à 1400 g/l selon la revendication 1.

3. Utilisation de poudre phosphate dicalcique anhydre ayant une surface spécifique inférieure à 2 m²/g selon la revendication 1 ou 2.
